# EUROPÄISCHE PATENTSCHRIFT

(11) **EP 3 737 987 B1**
(45) Veröffentlichungstag und Bekanntmachung des Hinweises auf die Patenterteilung: **02.02.2022**
(21) Anmeldenummer: 18833424.7
(22) Anmeldetag: 20.12.2018
(51) Int. Cl.: A61B 1/05, G02B 27/64, A61B 1/00, G02B 23/24

(54) **VIDEOENDOSKOP**
VIDEOENDOSCOPE
ENDOSCOPE VIDÉO

(30) Priorität: 10.01.2018 DE 102018100481
(43) Veröffentlichungstag der Anmeldung: 18.11.2020
(73) Patentinhaber: OLYMPUS Winter & Ibe GmbH, 22045 Hamburg (DE)
(72) Erfinder: WIETERS, Martin, 22885 Barbüttel (DE)
(74) Vertreter: Noack, Andreas
(86) Internationale Anmeldenummer: PCT/EP2018/086259
(87) Internationale Veröffentlichungsnummer: WO 2019/137785

(56) Entgegenhaltungen:
- WO-A1-2013/007356
- DE-A1-102004 044 119
- US-A- 5 575 756

## Beschreibung

Die Erfindung betrifft ein Videoendoskop gemäß Anspruch 1 oder 2.

Videoendoskope werden in der Medizin verwendet, um schwer zugängliche Bereiche eines Patienten zu untersuchen und ggf. zu behandeln. Sie weisen dazu einen langgestreckten Schaft auf, dessen distales Ende zu der zu untersuchenden Stelle geführt wird.

Am distalen Ende des Schafts ist eine Videokamera angeordnet, deren Signal zum proximalen Ende des Schafts und dort aus dem Endoskop herausgeführt wird. Das Signal wird dann zu einem Monitor geleitet und dargestellt. Die Videokamera eines Videoendoskops umfasst ein Objektiv und einen Bildaufnehmer, welcher z.B. als CCD- oder CMOS-Chip ausgeführt sein kann.

Das Objektiv eines Videoendoskops ist oftmals so ausgestaltet, dass es eine von der Längsachse des Schafts abweichende Blickrichtung aufweist. Als Blickrichtung wird dabei die objektseitige optische Achse des Objektivs bezeichnet. Dazu kann das Objektiv eine Prismenbaugruppe umfassen. Entsprechende Videoendoskope werden auch als ,seitlich blickende' Videoendoskope bezeichnet.

Seitlich blickende Videoendoskop erlauben es, durch Drehen des Videoendoskops um die Längsachse des Schafts ein besonders großes Blickfeld abzudecken. Dabei tritt allerdings das Problem auf, dass sich bei dem Drehen des Videoendoskops der Bildaufnehmer mit dreht. Dies führt dazu, dass die Horizontlage des beispielsweise auf einem Monitor dargestellten Bildes verloren geht, wodurch ein benutzender Arzt die Orientierung auf dem Bild verlieren kann.

Um dies zu verhindern ist der Bildaufnehmer so in dem Videoendoskop aufgenommen, dass er um die Längsachse des Schafts drehbar ist. Wird nun das Videoendoskop gedreht, kann der Bildaufnehmer in die entgegengesetzte Richtung gedreht werden, so dass er seine ursprüngliche Orientierung beibehält. Dadurch bleibt auch die Horizontlage des Bildes erhalten.

Aus technischen Gründen ist dabei das Objektiv oftmals geteilt und setzt sich aus einer distalen Objektivbaugruppe, welche die Prismenbaugruppe beinhaltet, und aus einer proximalen Objektivbaugruppe zusammen. Die beiden Objektivbaugruppen sind gegeneinander drehbar, wobei die distale Objektivbaugruppe drehfest zum Schaft des Videoendoskops ist, während die proximale Objektivbaugruppe gemeinsam mit dem Bildaufnehmer relativ zu dem Schaft drehbar ist. Die distale und die proximale Objektivbaugruppe sind dann durch ein axial und radial wirkendes Drehlager verbunden.

Ein zur Drehung der Objektivbaugruppen gegeneinander benötigtes Drehmoment wird in der Regel am proximalen Ende des Videoendoskops eingebracht, beispielsweise über einen Drehring, und mittels eines Innenrohres auf eine Fassung übertragen, welche den Bildaufnehmer und ggf. die proximale Objektivbaugruppe aufnimmt. Die distale Objektivbaugruppe ist in einem Außenrohr angeordnet, welches das Innenrohr umgibt. Am proximalen Ende des Innenrohrs und des Außenrohrs sind diese üblicherweise durch ein Lager gegeneinander abgestützt. Das Lager ist dabei an einer Lagerhülse montiert, welche drehfest am proximalen Ende des Außenrohrs befestigt ist.

Die WO 2013/007356 A1 zeigt ein Videoendoskop mit einem Griff, einem langgestreckten Schaft, einem am distalen Ende des Schafts angeordneten Objektiv, und einem proximal von dem Objektiv angeordneten Bildaufnehmer. Ein Innenrohr dient zur Übertragung einer Drehbewegung von dem Griff auf den Bildaufnehmer.

Aus der US 5,575,756 ist eine Vorrichtung zur Kopplung von Relay-Linsen in einem Endoskop bekannt. Die Vorrichtung weist ein Rohr mit beidseitig angeordneten elastischen Zungen auf, welche jeweils in umlaufende Nuten an den zu verbindenden Relay-Linsen eingreifen.

Um eine Montage des Videoendoskops zu vereinfachen, und/oder das Drehlager von Trägheitskräften des Rohres und damit verbundener Komponenten zu entlasten, kann das

Innenrohr drehfest, aber axial verschiebbar, mit der proximalen Objektivbaugruppe verbunden sein.

Die Herstellung der drehfesten und ggf. axial verschiebbaren Verbindung zwischen dem Innenrohr und der proximalen Objektivbaugruppe bzw. zwischen dem Außenrohr und der Lagerhülse ist nicht ganz unproblematisch. Um eine leichtgängige Montierbarkeit und axiale Verschiebbarkeit zu gewährleisten, muss die Verbindung mit einer Spielpassung ausgeführt sein. Dies führt aber dazu, dass auch die Übertragung des Drehmoments nicht spielfrei erfolgen kann. Dadurch kann es im Betrieb des Videoendoskops zu leichten Schwankungen in der Horizontlage des Bildes kommen, was störend ist.

Die Aufgabe der Erfindung besteht daher darin, ein Videoendoskop bereitzustellen, welches hinsichtlich der beschriebenen Problematik verbessert ist.

Diese Aufgabe wird gemäß eines Aspekts der Erfindung gelöst durch ein Videoendoskop gemäß Anspruch 1.

Gemäß eines weiteren Aspekts der Erfindung wird die Aufgabe gelöst durch ein Videoendoskop gemäß Anspruch 2.

Die elastischen Vorsprünge der ersten Kopplungsabschnitte greifen jeweils elastisch in die Ausnehmungen der zweiten Kopplungsabschnitte, dadurch wird das Spiel der Kopplung deutlich reduziert. Gleichzeitig bleibt die axiale Verschiebbarkeit der Verbindung gewährleistet.

Unter einer Objektivbaugruppe wird im Sinne der Erfindung eine Baugruppe des Videoendoskops verstanden, welche wenigstens ein optisches Element des Objektivs umfasst. Dabei kann eine Objektivbaugruppe auch nicht-optische Elemente umfassen, wie z.B. mechanische Fassungen, elektromagnetische Aktuatoren, Lager, oder ähnliches. Aus fertigungstechnischen Gründen kann es vorteilhaft sein, die Ausnehmungen auf dem Kopplungsabschnitt der Fassung und/oder der Lagerhülse vorzusehen, während die Vorsprünge an dem Kopplungsabschnitt des Innenrohrs und/oder des Außenrohrs vorgesehen sind. Umgekehrte Ausführungen, bei welcher die Ausnehmungen an dem Kopplungsabschnitt des Innenrohrs und/oder des Außenrohrs und die Vorsprünge an dem Kopplungsabschnitt der Fassung und/oder der Lagerhülse vorgesehen sind, sind jedoch ebenfalls möglich.

Die oben genannte Aspekte der Erfindung können einzeln oder gemeinsam in einem Videoendoskop zur Anwendung kommen.

In einer möglichen Ausgestaltung der Erfindung können die elastischen Vorsprünge aus dem Innen- bzw. Außenrohr geschnittene Federzungen sein. In diesem Fall ist die Herstellung der entsprechenden Vorsprünge besonders einfach. Zum Herstellen der Federzunge wird eine offene Kontur in das Rohr geschnitten, beispielsweise mittels eines Laserstrahls. Dabei bleibt die ausgeschnittene Federzunge an ihrer Basis mit dem Rohr verbunden. Anschließend wird die Federzunge plastisch verformt, so dass sie eine von der Kontur des Rohrs abweichende Ruhelage hat.

Die Federzungen werden vorzugsweise so geschnitten, dass ihre Basis beim Zusammensetzen mit der Objektivbaugruppe bzw. der Lagerhülse zuerst in den objektivseitigen bzw. lagerhülsenseitigen Kopplungsabschnitt eintaucht. Dabei werden die Federzungen durch den objektivseitigen bzw. lagerhülsenseitigen Kopplungsabschnitt ausgelenkt und federn von selbst in die Ausnehmungen zurück.

Gemäß einer bevorzugten Ausführung der Erfindung können die Federzungen dabei eine trapezförmige Struktur aufweisen. Unter einer trapezförmigen Struktur wird dabei im Sinne der Erfindung eine Struktur verstanden, bei der sich die Breite einer Federzunge von einer Basis, an welcher die Federzunge mit dem Rohr verbunden ist, zu einer Spitze, welche gegen die Federkraft der Federzunge frei beweglich ist, reduziert.

Eine entsprechend ausgeführte Federzunge kann zunächst mit der Spitze in eine Ausnehmung eintauchen und wird dann durch die Federkraft der Federzunge so weit in Richtung der Ausnehmung gedrückt, bis die Breite der Federzunge an einer Kontaktstelle mit der Ausnehmung genau der Breite der Ausnehmung entspricht. Dadurch ist eine spielfreie Kopplung gewährleistet.

In einer möglichen Ausführungsform der Erfindung können sich die Anzahlen der Ausnehmungen und Vorsprünge entsprechen, so dass jeder Vorsprung in eine Ausnehmung eingreift. Für eine gleichmäßige Drehmomentübertragung sind dabei die Vorsprünge und Ausnehmungen gleichmäßig über den Umfang der Kopplungsabschnitte verteilt, beispielsweise in einer 2x180°-Anordnung oder in einer 3x120°-Anordnung.

In einer weiteren möglichen Ausführungsform der Erfindung kann die Anzahl der Vorsprünge größer sein als die Anzahl der Ausnehmungen. Dabei können in einer bevorzugten Weiterbildung der Erfindung die überzähligen Vorsprünge eine elektrische Kontaktierung zwischen dem Außenrohr und der Lagerhülse herstellen.

Dies ist insbesondere sinnvoll, wenn das Außenrohr und die Lagerhülse gleichzeitig als elektromagnetische Abschirmung fungieren. In diesem Fall ist eine gute elektrische Kontaktierung an der Verbindungsstelle erforderlich, welche allein durch einen kleinflächigen Kontakt zwischen den Vorsprüngen und den Ausnehmungen nicht sicher gewährleistet werden kann.

Unter einem Innenrohr ist im Sinne der Erfindung ein Rohr zu verstehen, welches innerhalb mindestens eines weiteren Rohres angeordnet ist. Dabei muss das Innenrohr nicht zwangsläufig ein innerstes Rohr des Videoendoskops sein. Das Innenrohr kann über eine magnetische Kupplung mit einem Drehring des Videoendoskops verbunden sein, über welchen die Ausrichtung des Bildwandlers bei einer Drehung des Videoendoskops konstant gehalten wird.

Unter einem Außenrohr ist im Sinne der Erfindung ein Rohr zu verstehen, innerhalb dessen mindestens ein weiteres Rohr angeordnet ist. Dabei muss das Außenrohr nicht zwangsläufig ein äußerstes Rohr des Videoendoskops sein. Das Außenrohr kann drehfest mit dem Schaft des Videoendoskops gekoppelt sein, und so bei einer Drehung des Videoendoskops um die Längsachse des Schafts die proximale Objektivbaugruppe entsprechend mitnehmen.

Die Erfindung wird nachfolgend anhand einiger exemplarischer Darstellungen näher erläutert. Diese Darstellungen sollen lediglich zum besseren Verständnis der Erfindung beitragen, und dabei den allgemeinen Erfindungsgedanken keinesfalls einschränken.

Es zeigen:
- Fig.1:: ein Videoendoskop,
- Fig.2:: den distalen Abschnitt eines Videoendoskops,
- Fig.3:: einen proximalen Abschnitt des Videoendoskops.
- Fig.4:: ein Rohr mit ausgeschnittenen Federzungen.

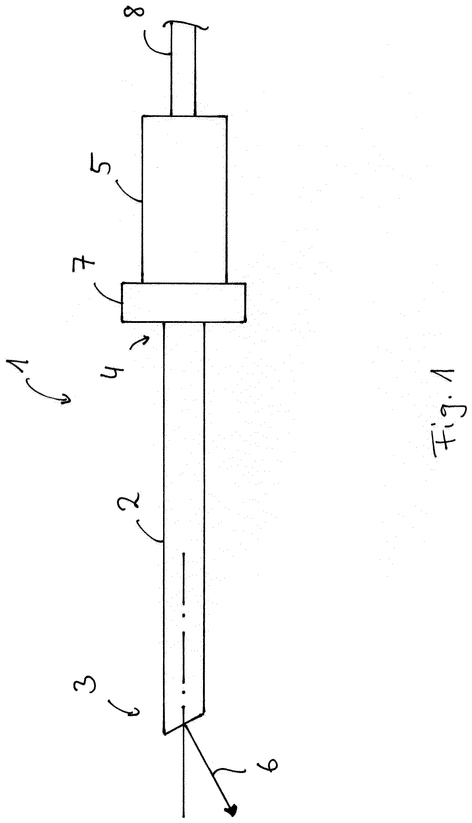

In Figur 1 ist ein Videoendoskop 1 dargestellt. Das Videoendoskop 1 weist einen langgestreckten Schaft 2 mit einem distalen Ende 3 und einem proximalen Ende 4 auf. Am proximalen Ende 4 des Schafts 2 ist ein Handgriff 5 angeordnet, mittels dessen das Videoendoskop 1 gehalten und bedient werden kann.

Am distalen Ende 3 des Schafts 2 ist ein nicht dargestelltes Objektiv angeordnet, dessen Blickrichtung in Richtung des Pfeils 6 ausgerichtet ist. Durch Drehen des Videoendoskops 1 kann die Blickrichtung des Objektivs um dessen Längsachse rotiert werden. Ein Drehring 7 dient dazu, die Horizontlage eines vom Videoendoskop 1 aufgenommenen Bildes zu kontrollieren. Die von dem Videoendoskop 1 erzeugten Videosignale werden über ein Kabel 8 ausgegeben.

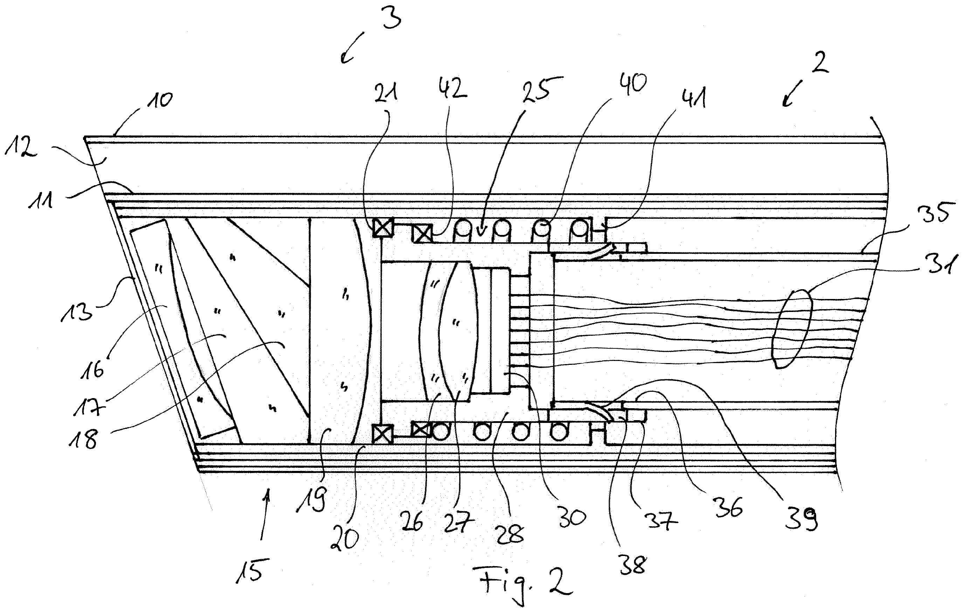

Der innere Aufbau des distalen Endes 3 des Schafts 2 ist in Figur 2 nähre dargestellt. Zwischen einem äußeren Hüllrohr 10 und einem exzentrisch in dem Hüllrohr 10 angeordneten Faserrohr 11 sind Lichtleitfasern 12 angeordnet, durch welche das Blickfeld des Videoendoskops 1 ausgeleuchtet werden kann. Das distale Ende des Faserrohrs 11 ist durch ein Fenster 13 verschlossen, welches hermetisch dicht in das Faserrohr 11 eingefügt ist.

Innerhalb des Faserrohrs ist dicht am Fenster 13 anliegend eine distale Objektivbaugruppe 15 vorgesehen, welche aus mehrere optischen Elementen 16,17,18,19 besteht, die in einem Außenrohr 20 gehalten werden. Bei den optischen Elementen 16,17,18,19 handelt es sich im dargestellten Beispiel um eine Meniskuslinse 16, zwei Prismen 17,18, und eine Positivlinse 19. Proximal von der distalen Objektivbaugruppe ist ein radial und axial wirkendes Lager 21 vorgesehen, an welchem sich eine proximaler Objektivbaugruppe 25 abstützt. Die proximale Objektivbaugruppe umfasst wiederum optische Elemente 26,27, welche in einer Fassung 28 aufgenommen sind. Bei den optischen Elementen 26,27 handelt es sich im dargestellten Beispiel um zwei Linsen, die gemeinsam einen Achromat bilden.

Die distale Objektivbaugruppe 15 und die proximale Objektivbaugruppe 25 bilden zusammen das Objektiv des Videoendoskops 1.

In der Fassung 28 ist weiterhin ein elektronischer Bildwandler 30 angeordnet. Dieser wandelt ein von dem Objektiv erzeugtes Bild in elektrische Signale um, welche über Leitungen 31 in Richtung des proximalen Endes des Videoendoskops 1 geleitet werden.

Um bei einer Drehung des Videoendoskops 1 um eine Längsachse des Schafts 2 die Horizontlage des Bildes konstant zu halten, kann die proximale Objektivbaugruppe 25 mit dem Bildaufnehmer relativ zu der distalen Objektivbaugruppe 15 verdreht werden. Dazu ist ein Innenrohr 35 drehfest und axial verschiebbar mit der Fassung 28 verbunden.

Das Innenrohr 35 greift mit einem Kopplungsabschnitt 36 koaxial in einen Kopplungsabschnitt 37 der Fassung 28 ein. Im Kopplungsabschnitt 37 der Fassung 28 sind Ausnehmungen 38 vorgesehen, in welche elastische Vorsprünge des Innenrohrs 35 eingreifen. Die elastischen Vorsprünge sind aus dem Innenrohr 35 ausgeschnittene Federzungen 39, welche nach dem Ausschneiden plastisch nach außen vorgebogen sind. Zum Zusammenbau des Innenrohrs 35 mit der Fassung 28 werden die Federzungen 39 elastisch nach innen gebogen und federn dann in die Ausnehmungen 38 zurück.

Alternativ zu der dargestellten Ausführung kann die Fassung 28 elastische Vorsprünge aufweisen, welche in Ausnehmungen des Innenrohrs 35 eingreifen.

Um eine spielfreie rotatorische Mitnahme zwischen dem Innenrohr 35 und der Fassung 28 zu gewährleisten, haben die Federzungen 39 einen trapezförmigen Querschnitt, verjüngen sich also von proximaler in distale Richtung. Dabei ist die maximale Breite der Federzungen 39 größer als die Breite der Ausnehmungen 38. Die Federzungen tauchen dann so weit in die Ausnehmungen 38 ein, bis sie spielfrei in diesen anliegen.

Eine Feder 40 drückt die Fassung 28 gegen das Lager 21, so dass kein axiales Spiel zwischen der distalen und der proximalen Objektivbaugruppe besteht. Dazu stützt sich die Feder 40 an einer Schulter 41 des Außenrohres ab. Zwischen der Feder 40 und der Fassung 28 kann ein weiteres Lager 42 vorgesehen sein. Bei der Lagern 21,42 handelt es sich vorzugsweise um keramische Gleitlager.

Die axiale Verschiebbarkeit des Innenrohrs 35 in der Fassung 28 verhindert, dass die Lager 21,42 durch axiale Trägheitskräfte belastet werden, welche ggf. über das Innenrohr 35 übertagen werden könnten.

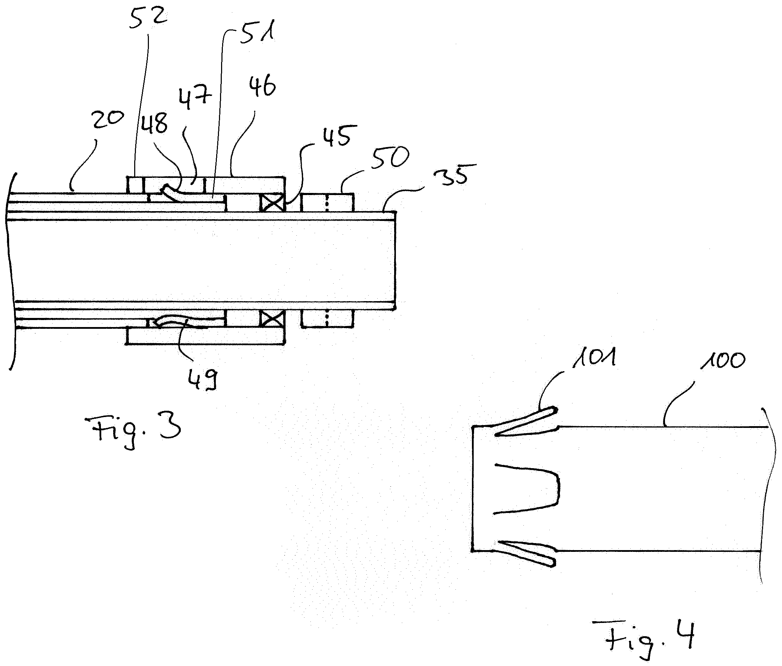

In Figur 3 ist ein proximaler Abschnitt des Videoendoskops ausschnittsweise 1 dargestellt. In diesem Abschnitt sind das Außenrohr 20 und das Innenrohr 35 durch ein radial wirkendes Lager 45 gegeneinander abgestützt.

Dabei ist das Lager 45 in einer Lagerhülse 46 befestigt, welche wiederum am proximalen Ende des Außenrohrs 20 befestigt ist. Die Befestigung der Lagerhülse 46 am Außenrohr 20 erfolgt analog zu der Befestigung der Fassung 28 am Innenrohr 35. Dazu weist die Lagerhülse 46 Ausnehmungen 47 auf, in welche elastische Vorsprünge des Außenrohrs 20 eingreifen. Die Vorsprünge des Außenrohrs sind im dargestellten Beispiel aus dem Außenrohr 20 ausgeschnittene Federzungen 48, welche plastisch nach außen vorgebogen sind. Zum Einsetzen des Außenrohrs 20 in die Lagerhülse 46 werden die Federzungen elastisch nach innen gebogen, so dass sie in die Ausnehmungen 47 zurückfedern. Für eine spielfreie Verbindung sind die Federzungen 48 ebenfalls trapezförmig ausgeführt.

Der Überlappungsbereich des Außenrohres 20 mit der Lagerhülse 46 bildet jeweilige Kopplungsabschnitte 51,52.

Alternativ zu der dargestellten Ausführung kann die Lagerhülse 46 elastische Vorsprünge aufweisen, welche in Ausnehmungen des Außenrohrs 20 eingreifen.

Im dargestellten Beispiel dient das Außenrohr 20 als elektromagnetische Abschirmung des Bildwandlers 30 sowie der Leitungen 31. Die Ableitung der Abschirmung erfolgt dabei über die Lagerhülse 46, daher ist ein guter elektrischer Kontakt zwischen dem Außenrohr 20 und der Lagerhülse 46 erforderlich. Dieser kann durch den kleinflächigen Kontakt der Federzungen 48 mit den Ausnehmungen 47 nicht gewährleistet werden.

Für die elektrische Kontaktierung sind aus dem Außenrohr 20 daher weitere Federzungen 49 ausgeschnitten, die ebenfalls plastisch nach außen vorgebogen sind. Diese Federzungen 49 greifen jedoch nicht in Ausnehmungen ein, sondern drücken gegen die innere Oberfläche der Lagerhülse 46. Dadurch wird auf einfache Weise ein sicherer Kontakt hergestellt.

Die Federzungen 49 und/oder die innere Oberfläche der Lagerhülse 46 können für eine bessere Kontaktierung vergoldet oder mit einem anderen kontaktfördernden Material beschichtet sein.

Proximal von dem Lager 45 sind Magnete 50 an dem Innenrohr angeordnet, welche Bestandteil einer nicht weiter dargestellten magnetischen Kupplung sind. Über die magnetische Kupplung kann das Innenrohr 35 gegen das Außenrohr 20 verdreht werden, beispielsweise mittels des Drehrings 7.

In Figur 4 ist ein Rohr 100 dargestellt, aus dessen Kontur Federzungen 101 geschnitten sind. Dabei kann es sich um ein Außenrohr oder ein Innenrohr handeln.

In den obigen Ausführungsbeispielen sind die Verbindungen so dargestellt, dass jeweils das Rohr in den Kopplungsabschnitt der Objektivbaugruppe bzw. der Lagerhülse eintaucht. Es ist natürlich auch möglich, das Rohr mit dem größeren Durchmesser zu versehen, so dass das Rohr die Objektivbaugruppe bzw. die Lagerhülse übergreift. In diesem Falle müssen die Federzungen natürlich nach innen vorgebogen sein.

## Patentansprüche

1. Videoendoskop mit
- einem langgestreckten Schaft (2) mit einem distalen Ende (3) und einem proximalen Ende (4), welcher ein Außenrohr (20) und ein Innenrohr (35) umfasst,
- einem am distalen Ende (3) des Schafts (2) angeordneten Objektiv, und
- wenigstens einem proximal von dem Objektiv angeordneten Bildaufnehmer (30), wobei
- das Objektiv eine distale Objektivbaugruppe (15) sowie eine proximale Objektivbaugruppe (25) aufweist,
- der Bildaufnehmer (30), ggf. gemeinsam mit der proximalen Objektivbaugruppe (25), gegenüber der distalen Objektivbaugruppe (15) um eine Längsachse des Schafts (2) verdrehbar ist,
- die distale Objektivbaugruppe (15) in dem Außenrohr (20) angeordnet ist, und
- ein Drehmoment zum Verdrehen des Bildaufnehmers (30) und ggf. der proximalen Objektivbaugruppe (25) gegenüber der distalen Objektivbaugruppe (15) durch das in dem Außenrohr (20) angeordnete Innenrohr (35) übertragen werden kann, welches drehfest und axial verschiebbar mit einer den Bildaufnehmer (30) und ggf. die proximale Objektivbaugruppe (25) aufnehmenden Fassung (28) des Videoendoskops verbunden ist, wobei
- das Innenrohr (35) und die Fassung (28) koaxial ineinandergreifende Kopplungsabschnitte (36,37) aufweisen, **dadurch gekennzeichnet, dass**
- ein erster der Kopplungsabschnitte (36,37) der Fassung (28) und des Innenrohrs (35) wenigstens zwei parallel zur Längsachse des Schafts (2) verlaufende Ausnehmungen (38) aufweist, und dass
- ein zweiter der Kopplungsabschnitte (36,37) der Fassung (28) und des Innenrohrs (35) wenigstens zwei elastische Vorsprünge (39) aufweist, welche sich in Richtung des ersten Kopplungsabschnitts (36,37) erstrecken und in die Ausnehmungen (38) eingreifen.

2. Videoendoskop mit
- einem langgestreckten Schaft (2) mit einem distalen Ende (3) und einem proximalen Ende (4), welcher ein Außenrohr (20) und ein Innenrohr (35) umfasst,
- einem am distalen Ende (3) des Schafts (2) angeordneten Objektiv, und
- wenigstens einem proximal von dem Objektiv angeordneten Bildaufnehmer (30), wobei
- das Objektiv eine distale Objektivbaugruppe (15) sowie eine proximale Objektivbaugruppe (25) aufweist,
- der Bildaufnehmer (30), ggf. gemeinsam mit der proximalen Objektivbaugruppe (25), gegenüber der distalen Objektivbaugruppe (15) um eine Längsachse des Schafts (2) verdrehbar ist,
- die distale Objektivbaugruppe (15) in dem Außenrohr (20) angeordnet ist,
- ein Drehmoment zum Verdrehen des Bildaufnehmers (30) und ggf. der proximalen Objektivbaugruppe (25) gegenüber der distalen Objektivbaugruppe (15) durch das in dem Außenrohr (20) angeordnete Innenrohr (35) übertragen werden kann, welches mit einer den Bildaufnehmer (30) und ggf. die proximale Objektivbaugruppe (25) aufnehmenden Fassung (28) des Videoendoskops verbunden ist, und
- das Außenrohr (20) und das Innenrohr (35) an ihrem proximalen Ende durch ein Lager (45) des Videoendoskops gegeneinander abgestützt sind, welches in einer am proximale Ende des Außenrohrs (20) befestigten Lagerhülse (46) des Videoendoskops angeordnet ist, wobei
- das Außenrohr (20) und die Lagerhülse (46) koaxial ineinandergreifende Kopplungsabschnitte (51,52) aufweisen, **dadurch gekennzeichnet, dass**
- ein erster der Kopplungsabschnitte (51,52) des Außenrohrs (20) und der Lagerhülse (46) wenigstens zwei parallel zur Längsachse des Schafts (2) verlaufende Ausnehmungen (47) aufweist, und ein zweiter der Kopplungsabschnitte (51,52) des Außenrohrs (20) und der Lagerhülse (46) wenigstens zwei elastische Vorsprünge aufweist, welche sich in Richtung des ersten Kopplungsabschnitts (51,52) erstrecken und in die Ausnehmungen (47) eingreifen.

3. Videoendoskop nach Anspruch 1 und 2.

4. Videoendoskop nach einem der Ansprüche 1 bis 3, **dadurch gekennzeichnet, dass** die elastischen Vorsprünge aus dem Innen- bzw. Außenrohr (20,35) geschnittene Federzungen (39,48,101) sind.

5. Videoendoskop nach Anspruch 4, **dadurch gekennzeichnet, dass** die Federzungen (39,48,101) eine trapezförmige Struktur aufweisen.

6. Videoendoskop nach einem der Ansprüche 1 bis 5, **dadurch gekennzeichnet, dass** sich die Anzahlen der Ausnehmungen (38,47) und der elastischen Vorsprünge entsprechen

7. Videoendoskop nach einem der Ansprüche 1 bis 5, **dadurch gekennzeichnet, dass** die Anzahl der elastischen Vorsprünge größer ist als die Anzahl der Ausnehmungen (38,47).

8. Videoendoskop nach einem der Ansprüche 2 oder 3 und nach Anspruch 7, **dadurch gekennzeichnet, dass** die überzähligen elastischen Vorsprünge eine elektrische Kontaktierung zwischen dem Außenrohr (20) und der Lagerhülse (46) herstellen.

## Claims

1. Videoendoscope comprising
- an elongated shaft (2) having a distal end (3) and a proximal end (4) and comprising an outer tube (20) and an inner tube (35),
- an objective lens disposed at the distal end (3) of the shaft (2), and
- at least one image sensor (30) disposed proximally of the objective lens, wherein
- the objective lens comprises a distal objective lens assembly (15), and a proximal objective lens assembly (25),
- the image sensor (30), optionally together with the proximal objective lens assembly (25), is rotatable relative to the distal objective lens assembly (15) about a longitudinal axis of the shaft (2),
- the distal objective lens assembly (15) is disposed in the outer tube (20), and
- a torque for rotating the image sensor (30) and optionally the proximal objective lens assembly (25) relative to the distal objective lens assembly (15) is transmittable through the inner tube (35) disposed in the outer tube (20), which inner tube (35) is non-rotatably and axially displaceably connected to a socket (28) of the video endoscope accommodating the image sensor (30) and optionally the proximal objective lens assembly (25)
wherein
- the inner tube (35) and the socket (28) comprise coaxially engaging coupling sections (36, 37),
**characterized in that**
- a first of the coupling sections (36, 37) of the socket (28) and of the inner tube (35) comprises at least two recesses (38) extending parallel to the longitudinal axis of the shaft (2), and **in that**
- a second of the coupling sections (36, 37) of the socket (28) and of the inner tube (35) comprises at least two elastic projections (39) which extend in the direction of the first coupling section (36, 37) and engage in the recesses (38).

2. Videoendoscope comprising
- an elongated shaft (2) having a distal end (3) and a proximal end (4) and comprising an outer tube (20) and an inner tube (35),
- an objective lens disposed at the distal end (3) of the shaft (2), and
- at least one image sensor (30) disposed proximally of the objective lens, wherein
- the objective lens comprises a distal objective lens assembly (15), and a proximal objective lens assembly (25),
- the image sensor (30), optionally together with the proximal objective lens assembly (25), is rotatable relative to the distal objective lens assembly (15) about a longitudinal axis of the shaft (2),
- the distal objective lens assembly (15) is disposed in the outer tube (20), and
- a torque for rotating the image sensor (30) and optionally the proximal objective lens assembly (25) relative to the distal objective lens assembly (15) is transmittable through the inner tube (35) disposed in the outer tube (20), which inner tube (35) is connected to a socket (28) of the video endoscope accommodating the image sensor (30) and optionally the proximal objective lens assembly (25)
- the outer tube (20) and the inner tube (35) are supported against each other at their proximal end by a bearing (45) of the video endoscope, which bearing is disposed in a bearing sleeve (46) of the video endoscope fastened to the proximal end of the outer tube (20),
wherein
- the outer tube (20) and the bearing sleeve (46) comprise coaxially engaging coupling sections (51, 52),
**characterized in that**
- a first of the coupling sections (51,52) of the outer tube (20) and of the bearing sleeve (46) comprises at least two recesses (47) extending parallel to the longitudinal axis of the shaft (2), and **in that**
a second of the coupling sections (51,52) of the outer tube (20) and of the bearing sleeve (46) comprises at least two elastic projections which extend in the direction of the first coupling section (51,52) and engage in the recesses (47).

3. Videodendoscope according to claim 1 and 2.

4. Videoendoscope according to any one of claims 1 to 3, **characterized in that** the elastic projections are spring tongues (39,48,101) cut from the inner or outer tube (20,35).

5. Videoendoscope according to claim 4, **characterized in that** the spring tongues (39,48,101) comprise a trapezoidal structure.

6. Videoendoscope according to any one of claims 1 to 5, **characterized in that** the numbers of the recesses (38,47) and of the elastic projections correspond to each other.

7. Videoendoscope according to any one of claims 1 to 5, **characterized in that** the number of elastic projections is greater than the number of recesses (38, 47).

8. Videoendoscope according to any one of claims 2 or 3 and according to claim 7, **characterized in that** the excess elastic protrusions establish an electrical contact between the outer tube (20) and the bearing sleeve (46).

## Revendications

1. Vidéoendoscope comprenant
- une tige allongée (2) avec une extrémité distale (3) et une extrémité proximale (4), qui comprend un tube extérieur (20) et un tube intérieur (35),
- un objectif disposé à l'extrémité distale (3) de la tige (2), et
- au moins un capteur d'image (30) disposé en position proximale par rapport à l'objectif,
où
- l'objectif comprend un assemblage d'objectif distal (15) ainsi qu'un assemblage d'objectif proximal (25),
- le capteur d'image (30), le cas échéant conjointement avec le module d'objectif proximal (25), peut tourner par rapport au module d'objectif distal (15) autour d'un axe longitudinal de la tige (2),
- l'assemblage d'objectif distal (15) est disposé dans le tube extérieur (20), et
- un couple de rotation pour faire tourner le capteur d'image (30) et, le cas échéant, l'assemblage d'objectif proximal (25) par rapport à l'assemblage d'objectif distal (15) peut être transmis par le tube intérieur (35) disposé dans le tube extérieur (20), lequel est relié de manière solidaire en rotation et mobile axialement à une monture (28) du vidéoendoscope recevant le capteur d'image (30) et, le cas échéant, l'assemblage d'objectif distal (25)
où
- le tube intérieur (35) et la monture (28) présentent des sections de couplage (36, 37) s'engageant coaxialement l'une dans l'autre,
**caractérisé en ce que**
- une première des sections de couplage (36, 37) de la monture (28) et du tube intérieur (35) présente au moins deux évidements (38) s'étendant parallèlement à l'axe longitudinal de la tige (2), et **en ce que**
- une deuxième des sections de couplage (36, 37) de la monture (28) et du tube intérieur (35) présente au moins deux saillies élastiques (39) qui s'étendent en direction de la première section de couplage (36, 37) et s'engagent dans les évidements (38).

2. Vidéoendoscope comprenant
- une tige allongée (2) avec une extrémité distale (3) et une extrémité proximale (4), qui comprend un tube extérieur (20) et un tube intérieur (35),
- un objectif disposé à l'extrémité distale (3) de la tige (2), et
- au moins un capteur d'image (30) disposé en position proximale par rapport à l'objectif,
où
- l'objectif comprend un assemblage d'objectif distal (15) ainsi qu'un assemblage d'objectif proximal (25),
- le capteur d'image (30), le cas échéant conjointement avec le module d'objectif proximal (25), peut tourner par rapport au module d'objectif distal (15) autour d'un axe longitudinal de la tige (2),
- l'assemblage d'objectif distal (15) est disposé dans le tube extérieur (20), et
- un couple de rotation pour faire tourner le capteur d'image (30) et, le cas échéant, l'assemblage d'objectif proximal (25) par rapport à l'assemblage d'objectif distal (15) peut être transmis par le tube intérieur (35) disposé dans le tube extérieur (20), lequel est relié à une monture (28) du vidéoendoscope recevant le capteur d'image (30) et, le cas échéant, l'assemblage d'objectif distal (25)
- le tube extérieur (20) et le tube intérieur (35) sont soutenus l'un contre l'autre à leur extrémité proximale par un palier (45) du vidéoendoscope, qui est disposé dans un manchon de palier (46) du vidéoendoscope fixé à l'extrémité proximale du tube extérieur (20),
où
- le tube extérieur (20) et le manchon de palier (46) comprennent des sections de couplage (51, 52) s'engageant coaxialement l'une dans l'autre,
**caractérisé en ce que**
- une première des sections de couplage (51, 52) du tube extérieur (20) et du manchon de palier (46) présente au moins deux évidements (47) s'étendant parallèlement à l'axe longitudinal de la tige (2), et
une deuxième des sections de couplage (51, 52) du tube extérieur (20) et du manchon de palier (46) présente au moins deux saillies élastiques qui s'étendent en direction de la première section de couplage (51, 52) et s'engagent dans les évidements (47).

3. Vidéoendoscope selon les revendications 1 et 2.

4. Vidéoendoscope selon l'une quelconque des revendications 1 à 3, **caractérisé en ce que** les saillies élastiques sont des languettes élastiques (39, 48, 101) découpées des tubes intérieur et extérieur (20, 35) respectivement.

5. Vidéoendoscope selon la revendication 4, **caractérisé en ce que** les languettes élastiques (39, 48, 101) présentent une structure trapézoïdale.

6. Vidéoendoscope selon l'une quelconque des revendications 1 à 5, **caractérisé en ce que** les nombres d'évidements (38, 47) et de saillies élastiques se correspondent.

7. Vidéoendoscope selon l'une quelconque des revendications 1 à 5, **caractérisé en ce que** le nombre de saillies élastiques est supérieur au nombre d'évidements (38, 47).

8. Vidéoendoscope selon l'une quelconque des revendications 2 ou 3 et selon la revendication 7, **caractérisé en ce que** les saillies élastiques surnuméraires établissent un contact électrique entre le tube extérieur (20) et le manchon de palier (46).
